# EUROPEAN PATENT APPLICATION

(11) **EP 1 437 403 A1**
(43) Date of publication of application: **14.07.2004**
(21) Application number: 02765389.8
(22) Date of filing: 30.08.2002
(51) Int. Cl.: C12N 15/02, C12N 5/06, C12Q 1/02, A61L 27/00

(54) **METHOD OF SCREENING REPORGRAMMING FACTOR, REPROGRAMMING FACTOR SCREENED BY THE METHOD, METHOD OF USING THE REPROGRAMMING FACTOR, METHOD OF DIFFERENTIATING UNDIFFERENTIATED FUSED CELLS AND METHOD OF CONSTRUCTING CELL, TISSUES AND ORGANS**

(30) Priority: 21.09.2001 JP 2001290101
(71) Applicant: Japan Science and Technology Corporation, Kawaguchi-City, Saitama Pref. 332-0012 (JP); Japan as Represented by the President of the University of Kyoto, Kyoto-shi, Kyoto 606-8501 (JP)
(72) Inventor: NAKATSUJI, Norio, Kyoto-shi, Kyoto 606-8507 (JP); TADA, Masako, Kyoto-shi, Kyoto 606-8507 (JP); TADA, Takashi, Kyoto-shi, Kyoto 606-8507 (JP)
(74) Representative: Holliday, Louise Caroline
(86) International application number: PCT/JP2002/008825
(87) International publication number: WO 2003/027277

(57) **Abstract**

By exposing somatic cells to a component derived from ES cells to act on somatic cells and detecting the activity thereof, the reprogramming agent can be screened. Further, by exposing somatic cells to a component including a reprogramming agent or an isolated reprogramming agent, the somatic cells can be reprogrammed. Furthermore, according to the present invention, since the tetraploid cells have proliferating capability and pluripotency, such cells can be differentiated and the cells, tissues or organs, which can be used for transplantation, can be produced.

SEQUENCE LISTING

<110> JAPAN SCIENCE AND TECHNOLOGY CORPORATION
JAPAN AS REPRESENTED BY THE PRESIDENT OF THE UNIVERSITY OF
KYOTO
NAKATSUJI, Norio
TADA, Masako
TADA, Takashi

<120> SCREENING METHOD FOR A REPROGRAMMING AGENT, REPROGRAMMING AGENT
SCREENED BY THE METHOD, METHOD FOR USING THE REPROGRAMMING AGENT,
METHOD FOR DIFFERENTIATING AN UNDIFFERENTIATED FUSION CELL, AND
PRODUCTION METHOD OF CELLS, TISSUES AND ORGANS

<130> TR011PCT

<140> PCT/JP02/08825
<141> 2002-08-30

<150> JP 2001-290101
<151> 2001-09-21

<160> 14

<170> PatentIn version 3.2

<210> 1
<211> 20
<212> DNA
<213> Artificial

<220>
<223> primer

<400> 1
ctaggtgagc cgtctttcca 20

<210> 2
<211> 20
<212> DNA
<213> Artificial

<220>
<223> primer

<400> 2
ttcagggtca gcttgccgta 20

<210> 3
<211> 24
<212> DNA
<213> Artificial

<220>
<223> primer

<400> 3
gtaggcacct gtggggaaga aact 24

<210> 4
<211> 25
<212> DNA
<213> Artificial

<220>
<223>  primer

<400>  4
tgagagctgt ctcctactat cgatt                                          25

<210>  5
<211>  27
<212>  DNA
<213>  Artificial

<220>
<223>  primer

<400>  5
acaagcttca aagcacaatg cctggct                                        27

<210>  6
<211>  30
<212>  DNA
<213>  Artificial

<220>
<223>  primer

<400>  6
gggtctagac tctcagccgg ctccctcagg                                     30

<210>  7

<211>  24
<212>  DNA
<213>  Artificial

<220>
<223>  primer

<400>  7
ctcggatcct acttctagct ttct                                                24

<210>  8
<211>  20
<212>  DNA
<213>  Artificial

<220>
<223>  primer

<400>  8
aaataccttg tgaaaacctg                                                     20

<210>  9
<211>  20
<212>  DNA
<213>  Artificial

<220>
<223>  primer

<400>  9

cagatccttg cagttcatcc 20

<210> 10
<211> 20
<212> DNA
<213> Artificial

<220>
<223> primer

<400> 10
tccacagtca cttgggttcc 20

<210> 11
<211> 23
<212> DNA
<213> Artificial

<220>
<223> probe

<400> 11
tttccctccc ggagattccc taa 23

<210> 12
<211> 29
<212> DNA
<213> Artificial

<220>

<223>  probe

<400>  12
cctgaggaga cggtgactga ggttccttg                                          29

<210>  13
<211>  25
<212>  DNA
<213>  Artificial

<220>
<223>  primer

<400>  13
aatcgcatta aaaccctccg aacct                                              25

<210>  14
<211>  24
<212>  DNA
<213>  Artificial

<220>
<223>  primer

<400>  14
tagcacaagt ggaattgtgc tgcg                                               24

## Description

### TECHNICAL FIELD

The present invention relates to a method for screening for an agent which reprograms a somatic cell nucleus which includes the steps of exposing a somatic cell to a component derived from embryonic stem cells, detecting an activity of the component which reprograms the somatic cells, and selecting the component having the reprogramming activity, a reprogramming agent obtained by the method, and a method for reprogramming somatic cells using the agent. The present invention also relates to a method for reprogramming somatic cells by using the agent, and then differentiating the reprogrammed cells to obtain cells, tissues or organs. Further, the present invention relates to a method for producing undifferentiated fusion cells of embryonic stem (ES) cells and somatic cells and differentiating the cells to obtain cells, tissues or organs, and the cells, tissues or organs obtained by the method.

### BACKGROUND ART

An embryonic stem (ES) cell is an undifferentiated totipotent cell which is induced from an embryo in an early stage and grows rapidly and has similar properties as those of an embryonic tumor cell. ES cells were first established by culturing an inner cell mass (ICM) of a mouse blastocyst on a feeder cell layer of mouse fibroblasts. ES cells have infinite lifetime under the conditions that undifferentiated states thereof are maintained in the presence of the feeder cell layer and/or leukemia inhibiting factor (LIF) [R.Williams et al., *Nature 336* :684-687(1988)]. Further, ES cells are known to have a high *in vitro* differentiating capability and can be differentiated into various types of cells by only culturing as an aggregate mass. ES cells are established from embryos at a stage before implantation and have pluripotency to be differentiated into various cell types derived from 3 germ layers, i.e., ectoderm, mesoderm and endoderm [M. J. Evans and M. H. Kaufman, *Nature 292:* 154-156 (1981); G. R. Martin, *Proc. Natl. Acad. Sci. USA. 78*: 7634-7638 (1981)]. More specifically, ES cells are capable of differentiating into any mature cell of an adult, and, for example, ES cells can be differentiated into both somatic cells and germ cells of a chimera animal by being introduced into a normal embryo at an early stage to form a chimeric embryo [R. L. Brinster, *J. Exp. Med. 140:* 1949-1956 (1974) : A. Bradley et al., *Nature 309:* 255-256 (1984)]. By mating chimera animals having cells derived from the ES cells introduced into germ cells such as testis, ovary, and the like, offspring composed of only cells derived from ES cells can be obtained. This means that animals can be produced with an artificially controllable genetic predisposition. With such an animal, it is possible to research a mechanism of growth and differentiation not only *in vitro* but also at an individual level. Unlike embryonic tumor cells, many of ES cells are normal cells with the normal diploid karyotype maintained, have a high rate of chimera formation, and a high probability of differentiation into cells of germ line [A. Bredley et al., *Nature 309:* 255-256 (1986)]. Thus, the scope of use of the ES cells is spreading outside the field of embryology.

For example, ES cells are the particularly useful in research on cells and on genes which control cell differentiation. For example, for functional analysis of genes having a known sequence, mouse ES cells have been used for a production of a mouse strain with a disrupted gene, introduced by genetic modification. The use of undifferentiated ES cells may be efficient and effective in functional analysis work after human genome analysis. Since ES cells can be differentiated into a wide variety of cell types *in vitro,* ES cells have been used for research on cell differentiationmechanisms in embryogenesis. It is becoming possible to induce the ES cells to differentiate into clinically advantageous cells, such as hematopoietic cells, cardiac muscle cells, and neurons of certain types by adding growth factors or forming germ layers [M. Wiles et al., *Development 111:* 259-267 (1991); W. Miller-Hance et al., *J. Biol. Chem. 268:* 25244-25252 (1993); V. A. Maltsev et al., *Mech. Dev. 44*: 41-50 (1993); G. Bain et al., *Dev. Biol. 168:* 342-357 (1995)]. Attempts to induce mouse SE cells to differentiate into advantageous cells have succeeded in the production of hematopoietic cells, cardiac muscle cells, specific neurons, and blood vessels [T. Nakano et al., *Science 265*:1098-1101(1994); R.Pacacios et al., *Proc. Natl. Acad. Sci. USA 92:* 7530-7534 (1995): V. A. Maltsev et al., *Mech. Dev*. *44*: 41-50 (1993); S. H. Lee et al., *Nat. Biotechnol. 18*: 675-679 (1999); H. Kawasaki et al., *Neuron 28*:31-40 (2000); S.-I. Nishikawa, *Development 125:* 1747-1757 (1998); M. Hirashima et al., *Blood 93*:1253-1263(1999)].

Currently, ES cells are established for the following animals: hamster [Doetshman T. et al., *Dev. Biol. 127*: 224-227 (1988)], pig [Evans M. J. et al., *Theriogenology 33:* 125128 (1990): Piedrahita J. A. et al., *Theriogenology 34*: 879-891 (1990); Notarianni E. et al., *J. Reprod. Fert. 40:* 51-56 (1990); Talbot N. C. et al., *Cell. Dev. Biol. 29A:* 546-554 (1993)]; sheep [Notarianni E. et al., *J. Reprod. Fert. Suppl. 43*: 255-260 (1991)]; bovine [Evans M. J. et al., *Theriogenology 33:* 125-128(1990); Saito S. et al., *Roux. Arch. Dev. Biol. 201:* 134-141(1992)]; mink [Sukoyan M. A. et al., *Mol. Reorod. Dev. 33:* 418-431 (1993)]; rabbit [Japanese National Phase PCT Laid-Open Publication No. 2000-508919]; and primates such as rhesus monkey, marmoset and the like [Thomson J. A. et al., *Proc. Natl. Acad. Sci. USA 92:* 7844-7848 (1995); Thomson J. A. et al., *Biol. Reprod. 55:* 254-259 (1996)]. Human ES cells are also established, and they show differentiating capability similar to those of mouse ES cells [J. A. Thomson et al., *Science 282:* 1145-1147 (1998); J. A. Thomson et al., *Dev. Biol. 38:* 133-165 (1998); B. E. Reubinoff et al., *Nat. Biotechnol. 18:* 399-404 (2000)]. It is expected that, by applying the enormous knowledge accumulating for differentiation induction and adjustment achieved by using mouse ES cells, human ES cells will become an infinite material for various cells and/or tissues for transplantation therapy for diseases including myocardial infarct, Parkinson's disease, diabetes, and leukemia and will solve the problem of a shortage of donors for transplantation therapy. In June 23, 2000, three research teams of Australia, the United States, and Germany reported in International Symposium on Stem Cell that they had succeeded in producing neuron and muscle cells from human ES cells for the first time. Further, a recent method for differentiating human ES cells into hematopoietic cell has been reported. However, even in the case where ES cells are used in transplantation therapy, the problem that immune rejection reaction occurs as in existing organ transplantation still remains.

It is now known that, by introducing a somatic cell nucleus into enucleated egg cells, the somatic cell nucleus is reprogrammed to be totipotent in mammal. In this way, clone sheep, bovine, mouse, pig and the like have been produced [Wilmut I. et al., *Nature 385*:810-813(1997); Kato Y. et al. , *Science 282*: 2095-2098 (1998); Wakayama T. et al., *Nature 394:* 369-374 (1998); Onishi A. et al. , *Science 289:* 1188-1190 (2000); Polejaeva I. A. et al., *Nature 407:* 86-90 (2000)]. By utilizing this technique, it is considered that it is possible to reprogram the nucleus of a somatic cell derived from a host which is to receive a transplant by using egg cells and producing a totipotent cell to produce a transplantation graft which does not cause immune rejection reaction. Further, with such a method of cell culturing, shortage of donors can be overcome. However, this method raises a problem with respect to ethical issues since it requires the use of egg cells.

In production of an animal clone using a somatic cell nucleus, the ratio of clones which survive to become adults is very low. The loss of embryos which occurs before transplantation may be partially due to lack of a nucleus - cytoplasm interaction [Kato Y. et al., *Science 282:* 2095-2098 (1998); Wakayama T. et al., *Nature 394:* 369-374 (1998)]. Further, a number of cloned fetuses are lost during pregnancy or immediately after birth. One of the reasons for these failures during the development stage is considered to be lack of effective reprogramming of the somatic cell nucleus. Allele specific methylation patterns in somatic cells have been examined for the *H19* and *IGF2r* gene loci. Normally, the allele-specific methylation is maintained in development after fertilization but not during germ cell development [Tremblay K. D. et al., *Nature Genet., 9:* 407-413(1995); Stoger R. et al., *Cell 73*:61-71(1993)]. In embryonic germ (EG) cell - thymocyte fusion cells, somatic cell methylation pattern of some imprinted genes including *Igf2r* is inhibited and both of alleles are not methylated [Tada M. et al., *EMBO J. 16:* 6510-6520 (1997)]. As a result, when EG cells are used, abnormal reprogramming of somatic cells may occur.

Many of the mechanisms related to epigenetic reprogramming of a somatic cell nucleus which guides normal embryo development are still to be examined. Recently, it was demonstrated that variation in the *ATRX* gene, which is a member of the SWI2/SNF2 helicase/ATPase family, changes the methylation profile of a sequence repeat in mammals [Gibbons R. J. et al., *Nat. Genet. 24:* 368-371 (2000)]. As a result, the possibility that demethylation occurs as a result of chromosome reconstruction has been suggested. It has also been reported that the maternal activity of ISWI which is a nucleosome dependent ATPase may function as a chromosome remodeller during a process of reprogramming the nucleus in cloned somatic cells of a frog [Kikyo N. et al., *Science 289:* 2360-2362 (2000)]. Nuclei extracted from *Xenopus* XTC-2 epithelial cells incubated for a short time period in an egg from *Xenopus* are reconstructed. Component TBP, which is an important part of a basic transcription complex, is lost.

It is considered that, once the reprogramming agent which guides normal reprogramming of the somatic cell nucleus is screened for, epigenetic operation will be possible by utilizing such agents. By screening such agents, cloning from adult somatic cells or production of tissue-specific stem cells without using an embryo of a mammal will also be possible. It is considered that such techniques will allow the production of donor cells for many clinical applications which require transplantation of cells or tissues.

### DISCLOSURE OF THE INVENTION

The objective of the present invention is to screen for an agent which can reprogram somatic cells. Further, the objective of the present invention is to provide cells, tissues and organs which can be used in the treatment of a number of diseases which requires transplantation of cells, tissues, and organs.

The present inventors fusedES cells and somatic cells to produce tetraploid cells and demonstrated that the resulting cells can proliferated *in vivo* or *in vitro,* the somatic cell nucleus is reprogrammed, andit has pluripotency. Based on these results, it is shown that ES cells produce an agent which performs normal reprogramming of the ES cell nucleus. It is considered that components included in ES cells act on somatic cells, the activity thereof is detected, and thus a reprogramming agent can be screened for. Further, it is considered that, by exposing somatic cells to a component including a reprogramming agent or an isolated reprogramming agent, the somatic cells can be reprogrammed.

Further, the present invention focuses on the fact that, since the tetraploid cells have proliferating capability and pluripotency, differentiation of such cells can produce cells, tissues or organs which can be used for transplantation. Particularly, it is considered that, in the future, by removing chromosomes derived from ES cells from the tetraploid cells to have undifferentiated cells which only have chromosomes derived from somatic cells, which are an ideal material for establishing cells, tissues, and organs which may be donor for treating various diseases. The present invention specifically relates to:
(1) A method for screening an agent which reprograms a somatic cell nucleus comprising exposing somatic cells to a component derived from embryonic stem (ES) cells, detecting an activity of the component which reprograms the somatic cells, and selecting the component having the reprogramming activity;
(2) A method according to item (1), wherein the somatic cells and/or the component derived from ES cells are cells or component derived from human;
(3) A reprogramming agent derived from ES cells;
(4) A method for reprogramming somatic cells using the reprogramming agent of item (3);
(5) A method for producing cells, tissues, or organs comprising exposing somatic cells to the reprogramming agent according to item (3) and reprogramming the somatic cells, and differentiating the reprogrammed cells;
(6) A method for producing cells, tissues, or organs comprising producing undifferentiated fusion cells of ES cells and somatic cells to differentiate the fusion cells;
(7) A method according to item (6), wherein the somatic cells are lymphocytes, spleen cells, or cells derived from testis;
(8) A method according to item (6) or (7), wherein the ES cells and/or the somatic cells are derived from human;
(9) Cells, tissues or organs obtained by a method according to any one of items (6)-(8); and
(10) Cells, tissues or organs according to item (9), the cells, tissues, or organs being used for transplantation.

The present invention relates to a method for screening for an agent which reprograms somatic cell nuclei. The method can be achieved by exposing appropriate somatic cells to a component derived from ES cells, detecting an activity of the component which reprograms the somatic cells, and selecting the component having the reprogramming activity. The somatic cells used herein may be, for example, lymphocytes, spleen cells, or cells derived from testis. The somatic cells are not limited to these cells and may be any cell as long as it has normal chromosomes, can be stably proliferated, and can be changed into undifferentiated cells. Particularly, it is preferable that the somatic cells are derived from the same species from which the ES cells which produce the component are derived (for example, in the case where the component derived from ES cells is derived from human, the somatic cells should be derived from human). It is also possible to use cell lines which have been already established.

As used herein, the term "reprogramming agent" or "agent which reprograms" refers to an agent which acts on cells to cause the cells to be in the undifferentiated state. As indicated in the examples below, ES cells cannot reprogram imprints in the nuclei of somatic cells, and can reprogram the epigenetic state of the nuclei of somatic cells so that germ cells can be developed. Therefore, it is clear that ES cells have an agent capable of reprogramming. Examples of an ES cell-derived component which is applied to somatic cells include, but are not limited to, components contained in ES cells, including cytoplasmic components, nuclear components, individual RNAs andproteins, and the like. When cytoplasmic or nuclear components including miscellaneous molecules are applied, the components may be fractioned to some degree with a commonly used technique (e.g., chromatography, etc. ) , and each fraction may be applied to somatic cells. If a specific fraction is revealed to contain a reprogramming agent, the fraction can be further purified so that a single molecule is eventually specified and such amolecule can be used. Alternatively, a fraction containing a reprogramming agent can be used without any purification to reprogram somatic cells. It may be considered that a single molecule achieves reprogramming. Alternatively, it may be considered that a plurality of molecules interact one another to alter somatic cells into the undifferentiated state. Therefore, the "reprogramming agent" of the present invention includes an agent consisting of a single molecule, an agent consisting of a plurality of molecules, and a composition comprising the single molecule or the plurality of molecules.

A reprogramming agent of the present invention can be screened for as follows . Components derived from ES cells are caused to act on somatic cells by means of contact, injection, or the like. The action is detected based on the expression of the Oct4-GFP marker gene, the activation of the X chromosome, or the like, as an indicator for reprogramming. A component having reprogramming activity is selected.

A "reprogramming agent contained in an ES cell" of the present invention can be obtained by a screening method as described above. There is a possibility that such a component is contained in cells other than ES cells. However, once a reprogramming agent is identified from an ES cell by the above-described method, such a reprogramming agent can be obtained or produced from other materials based on the identified reprogramming agent. For example, if a reprogramming agent obtained by the above-described method is RNA, the RNA can be sequenced and RNA having the same sequence can be synthesized using a well-known technique. Alternatively, if a reprogramming agent is a protein, antibodies for the protein are produced and the ability of the antibodies to the protein can be utilized to obtain the reprogramming agent from materials which contain the agent. Alternatively, the amino acid sequence of the protein is partially determined; a probe hybridizable to a gene encoding the partial amino acid sequence is produced; and cDNA and genomic DNA encoding the protein can be obtained by a hybridization technique. Such a gene can be amplified by PCR, though a primer needs to be prepared. A gene encoding a reprogramming agent obtained by any of the above-described methods can be used to produce the reprogramming agent by a well-known gene recombinant technique. Therefore, a "reprogramming agent contained in an ES cell" of the present invention is not necessarily obtained from ES cells. Therefore, the reprogramming agent includes all agents capable of reprogramming a somatic cell.

In a method for producing a cell, a tissue, or an organ from a somatic cell, an ES cell and/or an undifferentiated fusion cell of a somatic cell of the present invention, the cell is differentiated by a method which is not particularly limited as long as the cell is differentiated into a cell, a tissue or an organ, while the karyotype of the cell is substantially retained. For example, as shown in the examples, by introducing a cell into a blastocyst, subcutaneously injecting a cell into an animal (e. g. , a mouse, etc.) to form a teratoma, or the like, the cell can be differentiated into a cell, a tissue, and an organ. A desired cell, tissue, or organ can be isolated from the differentiated blastocyst or teratoma. A desired cell, tissue, or organ may be induced *in vitro* from a cell by adding a cell growth factor, a growth factor, or the like which is required for obtaining a cell of the type of interest. To date there have been reports for induction of blood vessel, neuron, muscle cell, hematopoietic cell, skin, bone, liver, pancreas, or the like from ES cells. These techniques can be applied when a cell, tissue, or organ corresponding to an implantation recipient is produced from a fusion cell according to the present invention.

When an ES cell is used in a method for producing a cell, a tissue, or an organ from an undifferentiated fusion cell according to the present invention, the ES cell can be established from an appropriate individual, or previously established ES cells derived from various organisms are preferably utilized. For example, examples of such a ES cell include, but are not limited to, ES cells of mouse, hamster, pig, sheep, bovine, mink, rabbit, primate (e.g., rhesus monkey, marmoset, human, etc.), and the like. Preferably, ES cells derived from the sample species as that of somatic cells of interest are employed.

Examples of somatic cells used in the method of the present invention for producing cells, tissues or organs from undifferentiated fusion cells according to the present invention, include, but are not particularly limited to, lymphocytes, spleen cells, testis-derived cells, and the like. Such somatic cells also include any somatic cell having a normal chromosome, which can be stably grown as a fusion cell and can be altered into an undifferentiated cell having pluripotency when it is fused with an ES cell. When cells, tissues or organs produced by the method are intended to be used for implantation, somatic cells obtained from transplantation individuals are preferably used.

As used herein, the term "fusion cell" refers to an undifferentiated cell which is produced by fusing an ES cell with a somatic cell as described above, can be stable grown, and has pluripotency. When chromosomes derived from a host ES cell are successfully removed from a fusion cell, the fusion cell can become a diploid undifferentiated cell which has somatic cell-derived chromosomes. The resultant cell is a preferable donor for more ideal treatment of various diseases. Examples of techniques for removing chromosomes derived from ES cells include irradiation, chemical treatment, methods using genetic manipulation, and the like. For example, by treating ES cells with irradiation or chemicals before fusion with somatic cells, it is possible to destroy only chromosomes derived from the ES cell after fusion. An exemplary chemical used in removal of chromosomes may be bromodeoxyuridine (BrdU). Chromosomes are treatedwith BrdU as follows: initially, ES cells are treated with this chemical; and UV irradiation is performed after fusing the ES cells with somatic cells. By irradiation, only chromosomes derived from the ES cell treated with BrdU are removed. A technique for removing chromosomes derived from an ES cell from a fusion cell by genetic manipulation may be conceived as follows. Initially, a LoxP sequence is randomly introduced into the genome of an ES cell. After fusing the ES cell with a somatic cell, the Cre protein is forcedly expressed so that only chromosomes derived from the ES cell are removed.

In the present invention, a method of fusing ES cells and somatic cells when producing cells, tissues, or organs from undifferentiated fusion cells is not particularly limited as long as ES cells and somatic cells are fused by contacting each other and form fusion cells. For example, as described in the examples, ES cells and somatic cells are mixed in a certain ratio, for example, in the case of producing fusion cells of ES cells and thymocyte, at 1:5, and then washed. The cells are suspended in an appropriate buffer such as mannitol buffer, and electrically fused. Besides such a high-voltage pulse cell fusion method utilizing structural changes in cell membrane by electrical stimulation (electroporation) [for example, EMBO J. 1: 841-845 (1982)], a cell fusion method using a chemical cell fusion acceleration substance such as Sendai virus, lysolecithin, glycerol, oleic acid ester, polyethyleneglycerol and the like is also known. The fusion method may be any fusion method as long as the cells formed by fusion of ES cells and somatic cells can stably proliferate as fusion cells and nuclei derived from somatic cells is reprogrammed such that the resulting cells are undifferentiated cells having pluripotency.

In the case where the cells, tissues, or organs of the present invention are used for transplantation, the cells, tissues, or organs may be used alone or may be used in combination with existing immunosuppression methods, such as immunosuppressants, surgical operations, or irradiation. Major immunosuppressants are adrenocorticosteroid, cyclosporine, FK506 and the like. Surgical operations may be, for example, extraction of lymph node, extraction of spleen, extraction of thymus, thoracic duct drainage, and the like. Irradiation maybe total body irradiation and transplantation graft irradiation. By combining these methods appropriately, the rejection reaction in the recipient against the transplantation graft can be more efficiently suppressed.

### BRIEF DESCRIPTION OF THE DRAWINGS

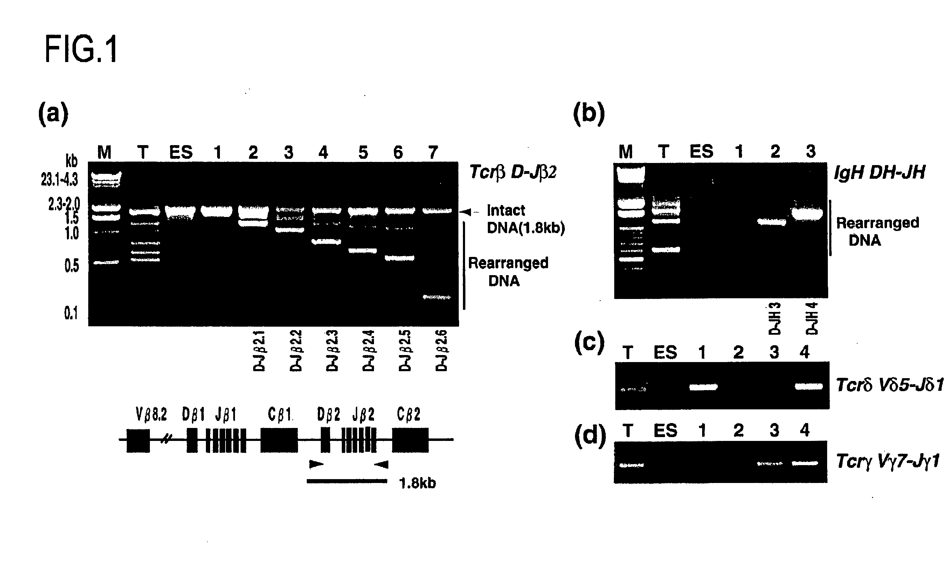

Figure 1 shows pictures showing the result of PCR analysis demonstrating DNA rearrangement of *Tcrβ, Tcrδ, Tcrγ* and *IgH* genes derived from thymocyte in ES fusion cells. Pictures (a) to (d) respectively show the results of PCR analysis using primer sets specific to the following regions: (a) D-J region of *Tcrβ;* (b) D-J region of *IgH;* (c) V-J region of *Tcrδ;* and (d) V-J region of Tcry. DNA samples used are as follows: T; derived from thymocytes from a (Rosa26 × Oct4-GFP) F1 mouse, ES; derived from ES cells, M; marker mixture of λ/HindIII DNA and an 100bp ladder DNA, 1 to 7; derived from ES hybrid clones.

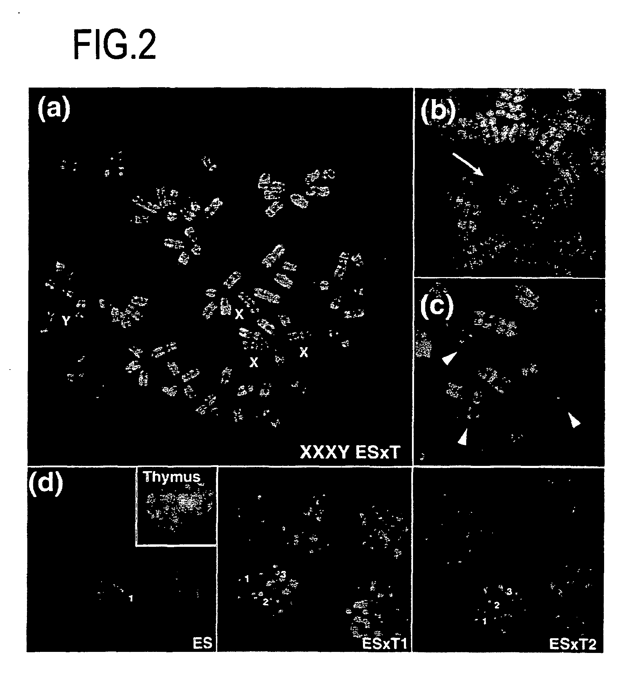

Figure 2 shows pictures showing reactivation of the X chromosome derived from thymocyte in ES fusion cells. (a) Results of R differential staining analysis at the time of replication of the X chromosome in ES fusion cells. In ES fusion cells, three X chromosomes (two X chromosomes from the female derived thymocyte, and one X chromosome from the male derived ES cell) are detected to be red and green, and are shown to be active. In (a), three X chromosomes replicated at the same time, and are shown enlarged in (c) (arrows). X chromosomes in female somatic cells (shown by arrows in (b)), and Y chromosome (in the middle of (a)) are uniformly stained red, and shown to be inactive. (d) *Xist* RNA is detected as spots of red signals on active X chromosome of male ES cell, while inactive X chromosome of female thymocyte is stained entirely giving a large red signal. In two ES hybrid cell lines (ES×T1 and ES×T2) examined, three spot red signals were detected for each nucleus.

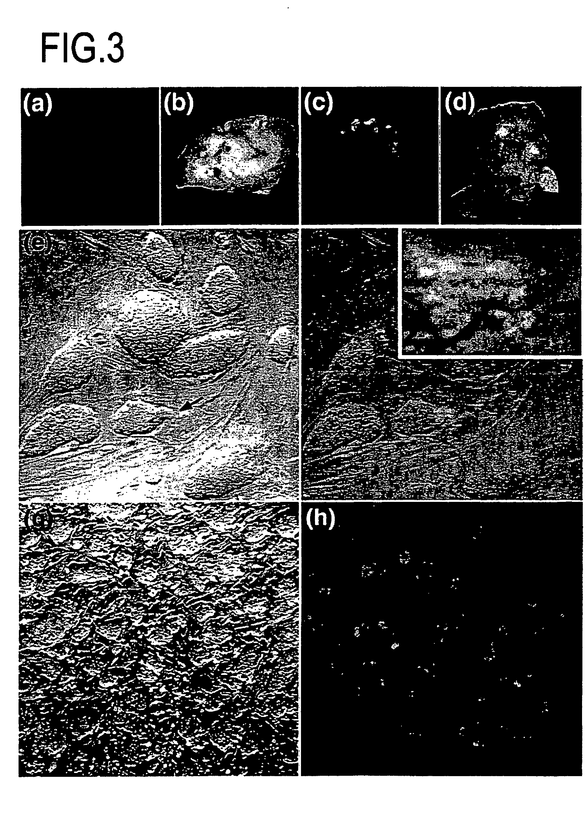

Figure 3 shows photomicrographs showing reactivation of ES fusion cells. GFP fluorescence images and bright field images are shown of thymus (a, b), and ovary (c, d) of (Rosa26 × Oct4-GFP) F1 mouse used for production of ES fusion cells. (e) Bright field images of colonies two days after fusion with the arrow indicating a GFP-positive colony as shown in (f). (f) GFP fluorescence image two days after fusion. The small GFP-positive colony amongst non-expressed ES cell colony. The picture of the positive colony is shown enlarged in an upper portion. (g) Bright-field image of (h). (h) Picture of a GFP-positive cell expanded from the colony after selection in G418.

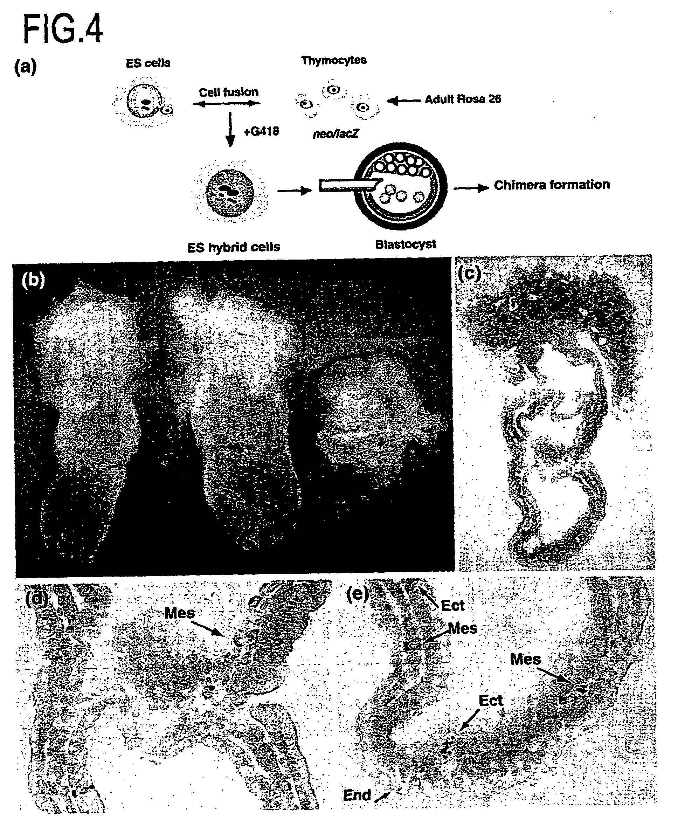

Figure 4 shows a diagram and pictures showing the development capability of ES fusion cells *in vivo.* (a) Schematic view showing a method of producing ES fusion cells and chimeric embryos, (b) Picture shows results of β-galactosidase active staining of E7.5 chimeric embryos having ES fusion cells. The cells derived from fusion cells are shown blue. (c) Picture showing results of histological analysis of a section of a E7.5 chimeric embryo which is sectioned along a longitudinal axis. (d, e) pictures showing chimeric embryo sections at higher magnification. Ect; ectoderm , Mes; mesoderm, and End; endoderm.

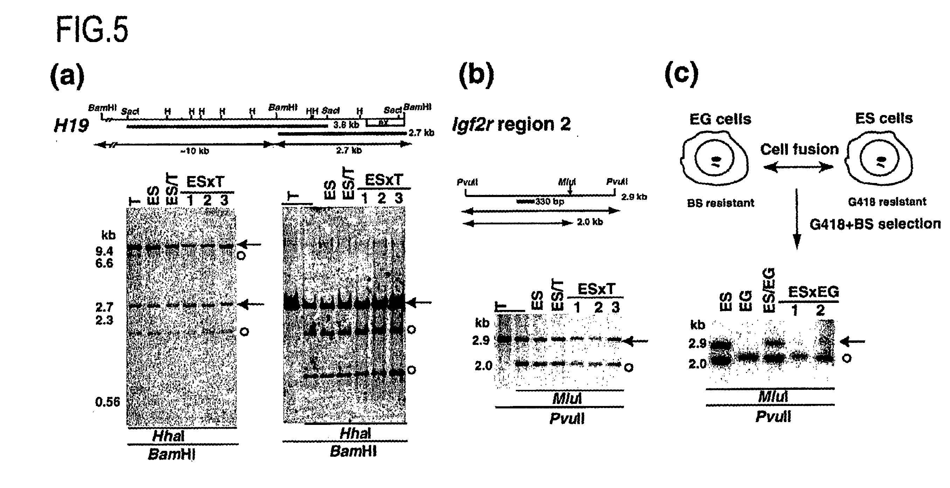

Figure 5 shows diagrams related to analysis of methylation of *H19* genes and *Igf2r* genes in ES hybrid and ES × EG fusion cells and pictures showing analysis results. (a): Analysis results for *H19* gene. (b) and (c): Analysis results for *Igf2r* gene. Arrows indicate methylated DNA fractions, and ○ represent unmethylated DNA fractions. A summary of experimentation methods is illustrated in (c). Abbreviations are as follows: T; thymocyte, ES/T; mixture of ES and thymocyte DNA at 1:1, ES/EG; mixture of ES and EG DNA at 1:1, ES×T; ES hybrid clone of ES cell and Rosa 26 thymocyte.

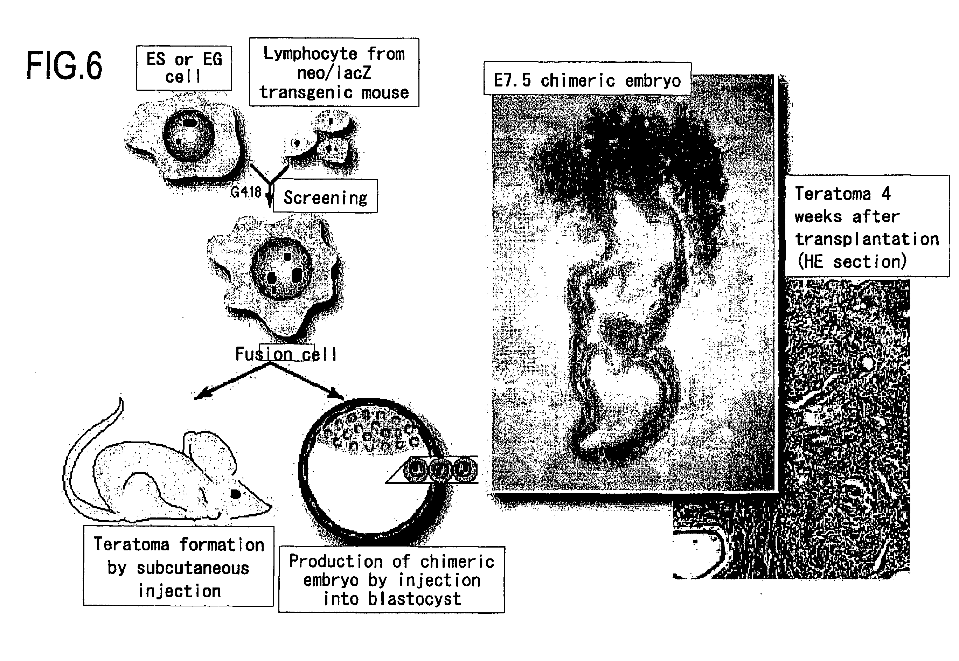

Figure 6 shows schematic views of teratoma formation and production of chimeric embryos and photomicrographs of chimeric embryos and teratoma.

### BEST MODE FOR CARRYING OUT THE INVENTION

Hereinafter, the present invention will be described by way of examples. The present invention is not limited to the examples.

### 1. Preparation of chimeric embryos

### (1) ES cell lines and EG cell lines

As ES cell lines, ES cell line TMAS-5 [Isolation, Culture, and Manipulation of embryonic stem cells (pp. 254-290), in "Manipulating the mouse embryo: A Laboratory Manual 2nd Edition" edited by Hogan, Beddington, Castantini and Lacy (Cold Spring Harbor Laboratory Press, USA)(1994)], and G418-resistant ES cell line NR-2 carrying a *neo*/*lacZ* reporter gene, which was derived from Rosa26 blastocyst [Friedrich G. and Soriano P., *Genes Dev. 5* : 1513-1523 (1991)], which were established from E3.5 male 129/Sv blastocysts, were used. As EG cell lines, EG cell line TMA-58G [Tada M. et al. , *EMBO J. 16*: 6510-6520 (1997) ], which was established from E12. 5 female PGC [Tada T. et al., *Dev. Gene. Evol. 207:* 551-561 (1998)], and blstoydine hydrochloride (BS) -resistant EG cell line (TMA-58G^{*bsr*}) , which was produced by transfecting a drug-resistant gene *pSV2bsr* into TMA-58G cells, were used. These cells were maintained on mouse G418-resistant primordial embryonic fibroblast (PEF) feeder cells (prepared using a typical technique from primary cultured fibroblasts in 12.5 day-old embryos of Rosa26), whichwere inactivatedwithmitomycin C in Dulbecco' s Modified Eagle's medium (DMEM) ) supplemented with ES medium (15% fetal bovine serum, 10⁻⁴ M2-mercaptoethanol, and 1000 units /mL recombinant leukemia inhibiting agent (LIF; ESGRO)). *TMA-58G*^{*bsr*} cells were cultured in ES medium containing 3 to 4 µG/mL BS. ES cell lines and EG cell lines, which were used in the cell fusion experiments below, were within passage number 10.

### (2) Preparation of hybrid clones by cell fusion

### (2)-1. ES fusion cells

Thymus cells derived from the following 3 types of 6 to 8 week-old mice:
(A) 129/Sv-TgR (Rosa26) 26Sor (referred to as Rosa26) [Friedrich G. and Soriano P., *Genes Dev. 5*:1513 -1523 (1991) ], which expresses a *neo*/*lacZ* reporter gene in cells of the whole the body;
(B) GOF-18/GFP (referred to as Oct4-GFP) [Yoshimizu T. et al., *Develp. Growth Differ., 41*:675-684 (1999)], whose totipotent and pluripotent cells specifically express GFP; and
(C) (Rosa26×Oct4-GFP) F1 transgenic mouse (hybrid mouse obtained by mating a female Rosa26 mouse with a male Oct4-GFP transgenic mouse [Yoshimizu T. et al., *Develop. Growth Differ., 41*:675-684 (1999)]), which contains both *a neo*/*lacZ* gene and an *Oct4-GFP* gene.

The Rosa26 mouse was identified by X-gal staining the tip of the tails thereof. The Oct4-GFP mouse was confirmed byPCR analysis of DNA from the tail thereof using the following primer: OCGOFU35, 5'-CTAGGTGAGCCGTCTTTCCA-3' (SEQ ID NO.: 1), and EGFPUS23, 5'-TTCAGGGTCAGCTTGCC GTA-3' (SEQ ID NO.: 2). A thymus obtained from the transgenic mouse was passed through a 18-gauge needle several times to obtain a suspension of single cells. As an ES cell, a TMAS-5 cell was used and mixed with the above-described 3 types of thymocytes at a ratio of 1: 5 (ES cell: thymocyte ) , followed by washing in PBS three times. The cells were suspended in 0. 3 M mannitol buffered solution at a concentration of 1×10⁶ cells/mL. A glass slide having a 1-mm electrode gap and Electro Cell Manipulator 2000 (BTX) were used to conduct electric fusion (E=2. 5 to 3.0 KV/cm) to prepare fusion cells. The fusion cells were cultured in ES medium for a day. InactivatedG418-resistant PEFs were screened for in ES medium containing 250 µg/mL G418 in 7 to 10 days. Fusion cell clones were collected and spread (passage number 1) in ES medium supplemented with G418, and were cultured for 3 to 4 days. ES hybrid clones were subcultured in new medium every two days.

### (2)-2, ES×EG fusion cell

ES×EG fusion cells were produced as follows. NR2 ES cells and TMA-58G^{*bsr*} EG cells were mixed at a ratio of 1:1, and were suspended in 0.3 M mannitol buffered solution at a concentration of 1×10⁶ cells/mL. ES×EG fusion cells were screened for in ES medium containing 250 µg/mL G418 and 3 to 4 µg/mL BS in 7 to 10 days.

ES hybrid clones could be maintained at a proportion (2.8×10⁻⁴) similar to that of EG hybrid clones produced by the present inventors in previous research [Tada M. et al., *EMBO J. 16*:6510-6520(1997)]. All types of fusion cells were similar to that of the parent ES cell, and no morphological change was found during culturing. Cytogenetic analysis using G-banding demonstrated a complete set of chromosomes including three X chromosomes and one Y chromosome in all of the 13 ES fusion cells and 2 ES×EG hybrid clones which were used in experimentation. Further, ES hybrid and ES×EG hybrid cell lines at passage number 2 to 4 were used in molecular analysis.

### (3) Confirmation of fusion

In order to confirm the fusion between ES cells and differentiated cells, 4 primer sets specific, respectively, to the D-J region of T cell receptor *(Tcr)* β, the D-J region of immunoglobulin *(Ig)* H, and the V-J regions of *Tcrδ* and *Tcrγ* were used to perform PCR amplification using DNA extracted fromthymocytes andES fusion cells as templates. For genomic DNA (0.5 µg) from an adult thymus, an ES cell and an ES hybrid clone, PCR amplification was performed to detect the rearrangement of each gene using the following primer sets:
(A) Dβ2-Jβ2 rearrangement of *Tcrβ* gene: Dβ2, 5'-GTAGGCACCTGTGGGGAAGAAACT-3'    (SEQ    ID    NO.: 3); Jβ2, 5'-TGAGAGCTGTCTCCTACTATCGATT-3' (SEQ ID NO.: 4) [Levin S. D. et al., *EMBO J. 12*: 1671-1680(1993)];
(B) D-J rearrangement of *IgH* gene: D μ, 5'-ACAAGCTTCAAAGCACAATGCCTGGCT-3' (SEQ ID NO.: 5); J μ, 5'-GGGTCTAGACTCTCAGCCGGCTCCCTCAGG-3' (SEQ ID NO.: 6) [Gu H. et al., *Cell 65:* 47-54(1991)];
(C) Vγ7-Jγ1 rearrangement of *Tcrγ* gene: Vγ7,5'-CTCGGATCCTACTTCTAGCTTTCT-3'   (SEQ   ID   NO.: 7); Jγ1, 5'-AAATACCTTGTGAAAACCTG-3' (SEQ ID NO.: 8) [Livak F. et al., *J. Immunol. 162*: 2575-2580(1999)]; and
(D) Vδ5-Jδ1 rearrangement of *Tcrδ* gene: Vδ5,5'-CAGATCCTTGCAGTTCATCC-3'    (SEQ    ID    NO.: 9); Jδ1,5'-TCCACAGTCACTTGGGTTCC-3' (SEQ ID NO.: 10) [Wilson A. et al., *Immunity 4:* 37-45(1996)].

PCR products were subjected to electrophoresis on 1. 2% agarose gel, followed by staining with ethidium bromide. The specificity of the PCR product was confirmed by Southern hybridization using: biotinylated Jβ2-specific oligoprobe (5'-TTTCCCTCCCGGAGATTCCCTAA-3' (SEO ID NO.: 11) [Levin S.D. et al., *EMBO J. 12*:1671-1680(1993)])for *Tcrβ*; and biotynylated JH4 oligoprobe (5'-CCTGAGGAGACGGTGACTGAGGTTCCTTG-3' (SEQ ID NO.: 12) [Ehlich A. et al., *Cell* 72:695-704(1993)]) for *IgH.*

The rearrangement of DNA is clear evidence indicating that a thymocyte has differentiated into a lymphoid cell [Fowlkes, B.J. and Pardoll D.M., *Adv. Immunol. 44*:207-264(1989)]. Rearrangement specific to *Tcr*β D-Jβ2.1, 2.2, 2.3, 2.4, 2.5 or 2.6 was observed in 45% of the hybrid clones (Figure 1a). Further, in several clones, similar rearrangement was observed in the D-J region of *IgH* (Figure **1b**), and the V-J regions of *Tcrδ* and *Tcrγ* (Figures **1c** and **1d**, respectively). Of the 31 ES hybrid clones studied, a total of 17 clones (55%) underwent rearrangement, which was, at least, researched. In these cases, it is considered that the ES cell were fused after differentiation of a thymocyte nucleus into a lymphoid cell.

### (4) X chromosome activity

In female somatic cells, one of the two X chromosomes is randomly inactivated due to dosage compensation of an X-linked gene. Inactivation of the X chromosome occurs in early cell division to induce the delay of transition of DNA replication into the late S phase, epigenetic changes including hypermethylation of DNA and low acetylation of histone H4 are known to occur. In cloned embryos obtained by nuclear transplantation of a somatic cell nucleus into an oocyte, the inactivated X chromosome of female somatic cells is reactivated [Eggan K. et al., *Science 290:* 1578-1581(2000)]. Therefore, activation of both X chromosomes serves as an indicator of the occurrence of reprogramming of a nucleus. In order to analyze the activity of X chromosomes, the present inventors studied using replication differential staining method [Sugawara O. et al., *Chromosoma 88*:133-138(1983)] (Figure **2a**).

### (4)-1. Timing of replication of X chromosome

Chromosome preparations of ES fusion cells and ES×EG fusion cells described in the above-described section (2) were produced by culturing the cells along with 150 µg/mL 5-bromo-2-deoxy uridine (BrdU) for 7 hours, where the cells were cultured for the last hour in the presence of 0.3 µg/mL colcemide. The cells were then subjected to hyposmotic treatment with 0.075M KCl at room temperature for 8 minutes. Thereafter, the cells were fixed by immersing in methanol: acetic acid (3:1) solution three times, followed by air drying. The cells were stained by freshly prepared acridine orange solution. The slide was observed under a fluorescence microscope with a standard B filter. After continued incorporation of BrdU at the late stage of the S period and acridine orange staining, active X chromosomes and autosomes were observed as red and green banded factors. Inactive X chromosomes were uniformly dark-red stained in female somatic cells due to the delay of replication (Figure 2b). Among all 32 cells (4n=80) whose karyotypes were determined, 6 fusion cell clones of an XY female ES cell and an XX female thymocyte carried three X chromosomes which were simultaneously replicated (Figure 2c).

### (4)-2. Xist RNA FISH

Probes prepared by nick translation of a mixture of Xist cDNA clones, which contained a series of exons 1 to 7, using cy3-dUTP (Amersham Pharmacia) [Sado T. et al., *Development 128*:1275-1286(2001)] were used and hybridized with chromosome preparations obtained as in 4-1. Hybridization and subsequent washing were performed as previously described [Lawrence J.B. et al., *Cell 57:* 493-502(1989)]. The result was in agreement with the result obtained in (4)-1, i.e., *, Xist* ( inactive X specific transcript ) RNA was not stably accumulated (spotted) on three X chromosomes in two ES hybrid cell lines tested by RNA FISH (fluorescence *in situ* hybridization) (Figure 2d). *Xist* accumulation was also instable on active X chromosomes of male ES cells, and was stable on inactive X chromosomes of female thymocytes (colored signal). Somatic cell nucleus-derived inactive X chromosomes acquired several properties of active X chromosomes after hybridization and had replication and *Xist* expression patterns similar to those observed in undifferentiated cells. Changes in replication timing and *Xist* RNA accumulation of X chromosomes of somatic cells in ES fusion cells shown in the above-described (4)-1 and (4) - 2 suggest that somatic cell nuclei were reprogrammed after cell fusion.

### (5) Reprogramming of somatic cell nucleus

A mouse line having an *Oct4-GFP* transgene was used to visualize the reprogramming of somatic cell nuclei (Figure 3). Expression of *Oct4* was specifically observed in germ cells, embryos before implantation, and ectoblasts of early embryos before implantation. Therefore, the activity of *Oct4* can be used as an ideal marker for identification of totipotency and/or pluripotent cells. The expression pattern of *Oct4-GFP* is known to be comparable to the expression pattern of endogenous *Oct4* [Yoshimizu T. et al., *Develop Growth Differ. 41*:675-684(1999)]. Expression of *Oct4-GFP* was examined for the thymus and ovary of *Oct4-GFP* transgenic mice. GFP was detected in the growing ovary, but not in the thymus (Figures **3a** to **d**).

Thymus cells of Oct4-GFP transgenic mice were fused with ES cells, followed by culturing without screening. Expression of GFP was examined every 12 hours. Expression of GFP in living ES fusion cells on a culture dish was investigated under a dissecting microscope (Leica) with a GFP exciting source and a GFP filter. After 48 hours, a GFP positive colony consisting of 16 cells was observed at the periphery of a larger non-expressing colony (Figures 3e, f). Subsequently, several other GFP positive colonies were observed on the same culture plate before reaching confluency. No GFP positive cells was observed among non-fusion thymocytes cultured under the same conditions. In order to investigate whether or not somatic cell nuclei can be reprogrammed in all ES fusion cells, thymocytes of G418 selection-resistant (Rosa26×Oct4-GFP) F1 mouse were used. After screening, 36 of the resultant 37 clones expressed GFP (97%). The expression was stably maintained even after subculturing over some passages (Figures **3g**, **h**), indicating that the thymocyte nucleus was reprogrammed in most of the ES fusion cells. The *Oct4-GFP* transgene, which was suppressed in thymocytes before cell fusion, was reactivated in the ES fusion cells. This further supports the result of (4) that after cell fusion, somatic cell nuclei were reprogrammed.

### (6) Introduction into blastocysts

Normal diploid blastocysts were used to produce chimeric embryos with ES fusion cells. Diploid blastocysts were obtained from the uteruses of Day 3.5 pregnancy ICR females mated with ICR males. Hybrid clones with the above-described (Rosa26×Oct4-GFP) F1 mouse-derived (differentiated) thymocytes and hybrid clones with Rosa26 mouse-derived thymocytes were used as tetraploid fusion cells. The tetraploid fusion cells were microinjected into the blastocoele pores of well expanded blastocysts (Figure **4a**). These blastocysts were transferred into the uteruses of pseudopregnant ICR females. Chimeric embryos were removed from the E7.5 uteruses, followed by removal of the Reichert membrane, and β-galactosidase staining and histological analysis.

### (6)-1. β-galactosidase active staining

By β-galactosidase active staining, the relative contribution of a fusion cell to each chimera was confirmed. Cultured cells were washed with PBS, and fixed using PBS containing 1% formaldehyde, 0.2% glutaraldehyde, 0.02% NP40 and 1 mMMgCl₂, at 4°C for 5 minutes. The same fixing solution was used to fix embryos and mouse tails at 4°C for 3 to 4 hours. The samples were washed with PBS, f ollowed by staining with a reaction mixture containing 1 mg/mL 4-Cl-5-Br-indolyl-β-galactosidase (X-gal) with dimethyl formamide, 5 mM potassium ferricyanide, 5 mM potassium ferricyanide, and 2 mM MgCl₂ in PBS at room temperature for 24 to 48 hours.

### (6)-2. Histological analysis

E7.5 embryos stained with X-gal were dehydrated with an ascending alcohol series, and embedded in JB-4 plastic resin (polyscience, Warrington, PA). Ultrathin sections (2-4 µm thick) were negative stained with 0.25% eosin Y. Four week old teratoma fixed and embedded in paraffin were cut into 8-µm thick sections. The serial sections were stained with hematoxylin and eosin.

As a result, 8 of 20 E7.5 embryos were positive, indicating the limited contribution of the fusion cell (Figures **4b**, **c**). Detailed analysis revealed derivatives from the fusion cell in the embryonic ectoderm, embryonic mesoderm, and the internal organ endoderm (Figures **4d**, **e**). As described above, the ES fusion cell had a developmental capability of differentiating into three primordial germinative layers (ectoderm, mesoderm, and endoderm) in early embryos before implantation.

### (7) Methylation of DNA

Next, DNA of thymocytes, ES cells and prepared hybrid clones was investigated by Southern blot hybridization as to whether or not the reprogramming of a thymocyte nucleus has an influence on the methylation of an imprinted genetic locus. Southern blot hybridization was performed by separating genomic DNA digested with a restriction enzyme into fractions using 0.8% agarose, transferring the fractions onto a Hybond N+membrane (Amersham) by alkali blotting, and hybridizing DNA with a ³²P-d CTP-labeled probe.

### (7)-1. H19 genetic locus

It is considered that a paternal methylated region is incorporated upstream of the maternal *H19* genetic locus to be expressed, so that primordial methylation imprinting is maintained [Tremblay K.D. et al., *Nature Genet. 9*:407-413(1995)]. DNA samples of thymocytes, ES cells, and prepared hybrid clones were digested with *BamHI* and a methylation sensitive restriction enzyme *HhaI.* A 3.8-kb *SacI* probe and a 2.7-kb BamHI probe were used to detect 10-kb nd2.7-kb paternal methylated fragments and 7.0-kb and 1.8-kb maternal unmethylated fragments in DNAs from both the thymocyte and the ES cell. The same pattern was found in the hybrid clone. There was no difference in the relative intensity (RI) of bands between the methylated (RI=0.60) and unmethylated fragment (RI=0.40) bands (Figure 5a). Similar results were observed using *BamHI* probes, in which 2.7-kb paternal methylated fragments and 1.8-kb and 0.8-kb maternal unmethylated fragments were identified. For all samples, methylated (RI=0.55) and unmethylated (RI=0.45) bands were similarly detected (Figure 5a).

### (7)-2. Igf2r genetic locus

Probes for analysis of methylation of Igf2r region 2 were produced by PCR using the following primers: 5'-AATCGCATTAAAACCCTCCGAACCT-3' (SEQ ID NO.: 13) and 5'-TAGCACAAGTGGAATTGTGCTGCG-3' (SEQ ID NO.: 14) [Stoger R. et al., *Cell 73*:61-71(1993)]. ACpG island, which is anintron of the *Ifg2r* gene and is known to be imprinted with methylation, is methylated only on an expressed allele [Stoger R. et al., *Cell 73*:61-71(1993)]. As in the above-described section (7)-1, each DNA sample was digested with *PvuII* and a methylation sensitive restriction enzyme *MluI*. With the 330-bp *Igf2r* CpG island probe, a 2.9-kb maternal methylated fragment and a 2.0-kb paternal unmethylated fragment were detected in DNAs from both the thymocyte and the ES cell (Figure **5b**). The same pattern was also found in the hybrid clone. There was no difference in relative intensity (RI) between methylated (RI=0.55) and unmethylated (RI=0.45) bands (Figure **5a**).

According to sections (7)-1 and (7)-2, it was demonstrated that the primordial methylation of the *H19* upstream region and the *Igf2r* intron region of the genome of a thymocyte are not affected by fusion with an ES cell. This results differs from previous observation of a hybrid clone of a thymocyte and an EG cell derived from a germ cell PGC of an E12.5 mouse in which the maternal specific methylation of *Igf2r* disappeared [Tada M. et al., *EMBO J. 16*: 6510-6520(1997)]. The maintenance of the somatic cell methylation pattern in ES fusion cells suggests that ES cells and EG cells have different control mechanism for regulating the DNA methylation of imprinted genes. While the maternal allele specific methylation of *Igf2r*was observed in ES cells, it was not observed in EG cells and a control 1:1 mixture of ES cell DNA and EG cell DNA at a ratio of about 1:3 (methylation/RI=0.27, unmethylation/RI=0.73). In the ES×EG hybrids, methylation bands disappeared (Figure **5c**), indicating that demethylation activity in EG cells is dominant over the maintenance of methylation imprinting in ES cells.

### 2. Production of teratoma

Fusion cells of TMAS-5ES cells and Rosa26-derived thymocytes, which were produced with a method similar to that for production of chimeric embryos in section 1, were used as tetraploid fusion cells. About 100 million to 500 million tetraploid fusion cells were subcutaneously injected into the posterior limb inguinal region of a SCID mouse (CLEA Japan KK). Four weeks after subcutaneous injection, teratoma was collected. By X-gal staining, it was determined that the teratoma was derived from the fusion cell. Thereafter, the teratoma was fixed in Bouin's fixative, followed by Haematoxyline-Eosin (HE) staining to stain both the nucleus and cytoplasm. As a result of HE staining, muscles, cartilages, epithelial cells, and neurons were observed (Figure 6).

### INDUSTRIAL APPLICABILITY

According to the present invention, an experimentation systemwhich can be operated so as to enable research on molecular mechanisms related to reprogramming is provided. This utilizes the capability of reprogramming at least a part of a somatic cell nucleus of ES cells *in vitro,* which is observed for the first time by producing fusion cells of ES cells and somatic cells. Unlike EG cells, ES cells cannot reprogram paternal implementation. Results of methylation analysis of *Igf2r* in ES × EG fusion cells suggest that EG cells have stronger additional dominance factors involved in epigenetic reprogramming. Actually, ES cells and EG cells respectively reflect original characteristics of the cells. Therefore, both the ES cells and EG cells can be advantageous materials for epigenetic reprogramming and screening for a factor involved in demethylation of early stage germ cells and germ line PGC.

By using a method for screening for an agent which reprograms somatic cell nuclei according to the invention, which is established based on the above-described observation, a reprogramming agent is obtained. Somatic cells are exposed to the reprogramming agent so that the somatic cells can be undifferentiated. Such undifferentiated cells derived from somatic cells have the same karyotype as that of the original somatic cell, and can be an ideal material for establishing cells, tissues, and organs which can be used as donors for treating various types of diseases.

The present inventors produced a tetraploid composed of fused ES cells and somatic cells and demonstrate that the cells can be proliferated *in vivo* or *in vitro,* the somatic cell nucleus is reprogrammed, and it has pluripotency. The tetraploids can be used in production of cells, tissues, and organs which may be used as donors for treating various diseases as ES cells. Furthermore, if chromosomes derived from host ES cells are successfully removed from such tetraploids , the cells become diploid undifferentiated cells which have only chromosomes derived from somatic cells. These cells may be more ideal donors for treating various diseases.

## Claims

1. A method for screening an agent which reprograms a somatic cell nucleus comprising:
exposing somatic cells to a component derived from embryonic stem (ES) cells;
detecting an activity of the component which reprograms the somatic cells; and
selecting the component having the reprogramming activity.

2. A method according to claim 1, wherein the somatic cells and/or the component derived from ES cells are cells or component derived from human.

3. A reprogramming agent derived from ES cells.

4. A method for reprogramming somatic cells using the reprogramming agent of claim 3.

5. A method for producing cells, tissues, or organs comprising:
exposing somatic cells to the reprogramming agent according to claim 3 and reprogramming the somatic cells; and
differentiating the reprogrammed cells.

6. Amethod for producing cells, tissues, or organs comprising producing undifferentiated fusion cells of ES cells and somatic cells and differentiating the fusion cells.

7. A method according to claim 6, wherein the somatic cells are lymphocytes, spleen cells, or cells derived from testis.

8. A method according to claim 6 or 7, wherein the ES cells and/or the somatic cells are derived from human.

9. Cells, tissues or organs obtained by a method according to any one of claims 6 through 8.

10. Cells, tissues or organs according to claim 9, the cells, tissues, or organs being used for transplantation.
